# FASCICULE DE BREVET EUROPEEN

(11) **EP 0 908 465 B1**
(45) Date de publication et mention de la délivrance du brevet: **18.07.2001**
(21) Numéro de dépôt: 98402417.4
(22) Date de dépôt: 01.10.1998
(51) Int. Cl.: C07H 3/06, C07H 3/02

(54) **Procédé de fabrication d'un aldose ou d'un dérivé d'aldose**
Verfahren zur Herstellung von Aldosen oder von Aldosederivaten
Process for the production of an aldose or of an aldose derivative

(30) Priorité: 07.10.1997 FR 9712477
(43) Date de publication de la demande: 14.04.1999
(73) Titulaire: Roquette Frères, 62136 Lestrem (FR)
(72) Inventeur: Tamion, Rodolphe, 62157 Allouagne (FR)
(74) Mandataire: Boulinguiez, Didier

(56) Documents cités:
- EP-A- 0 233 816
- EP-A- 0 716 067
- EP-A- 0 810 292
- EP-A- 0 829 485

## Description

La présente invention a pour objet un procédé de fabrication d'un aldose ou d'un dérivé d'aldose.

Plus précisément, la présente invention a pour objet un procédé de fabrication d'un aldose ou d'un dérivé d'aldose, à n atomes de carbone, à partir d'un sel d'un dérivé acide d'ose à n + 2 atomes de carbone comportant au moins un motif α-hydroxyacide et de peroxyde d'hydrogène, catalysé par un sel d'un métal choisi dans le groupe constitué du cobalt, du nickel et du ruthénium, et se déroulant en phase aqueuse.

Dans la présente invention, on entend par :
- "aldose", un ose comportant une fonction aldéhyde;
- "dérivé d'aldose", plus particulièrement un dialdose (ose comportant deux fonctions aldéhydes) ou un acide uronique (acide monocarboxylique dérivé d'un aldose par remplacement du groupement CH₂OH par un groupement COOH) ;
- "dérivé acide d'ose comportant au moins un motif α-hydroxyacide", un acide mono- ou dicarboxylique dérivé d'un aldose et comportant une fonction CHOH en α de la ou des fonction(s) acide(s).

En particulier, le procédé de la présente invention permet d'obtenir, avec un excellent rendement, un aldose à partir d'un acide aldonique, un dialdose à partir d'un acide uronique, ou un acide uronique à partir d'un acide aldarique.

Les aldoses obtenus par la mise en oeuvre du procédé conforme à l'invention sont d'un grand intérêt en tant que tels, mais seraient surtout des intermédiaires de synthèse fort importants s'il advenait que l'on puisse les produire én grande quantité et à faible coût. En effet, une étape complémentaire simple d'hydrogénation de ces aldoses permet d'obtenir facilement les alditols correspondants qui sont tous des polyols pouvant être employés dans de multiples applications et notamment en tant que substituts non cariogènes et hypocaloriques du saccharose.

Les dialdoses obtenus par la mise en oeuvre du procédé conforme à l'invention, en tant que composés polyhydroxylés à deux fonctions aldéhydes, sont également des intermédiaires de synthèse potentiellement importants notamment pour la fabrication d'acide aldarique par simple oxydation de fonctions aldéhydes.

Enfin, les acides uroniques obtenus par la mise en oeuvre du procédé conforme à l'invention, en tant qu'acides polyhydroxycarboxyliques, possèdent des propriétés séquestrantes pouvant être exploitées dans le domaine des ciments, mortiers ou ciments où de tels acides ont été proposés comme retardateurs de prise, ou encore dans le domaine de la détergence où ces acides ont été proposés pour le nettoyage d'articles en verre ou en métal ou à titre d'additifs pour détergents.

C'est en étudiant la méthode exposée par RUFF, il y après d'un siècle (Ber. 32, 3674 (1899) ; 33, 1799 (1900)) que la Demanderesse a mis au point un nouveau procédé de fabrication d'un aldose ou d'un dérivé d'aldose, par voie chimique, à partir d'un dérivé acide d'ose ou de ses sels.

La méthode de RUFF permet de passer, de façon générale, d'un acide aldonique comportant n carbones à un aldose comportant (n-1) carbones grâce à l'action combinée d'ions ferriques et d'eau oxygénée. Cependant, les rendements en aldose sont très modestes.

Ainsi, la conversion de l'acide gluconique en D-arabinose est réalisée selon cette méthode.

Quelques améliorations ont, par la suite, été apportées par R.C. Hockett et C.S. Hudson (J. Amer. Chem. Soc. 56, 1632-1633, (1934) et ibid. 72, 4546, (1950)) et par le document US-A 3.755.294. Des rendements de 60 % d'arabinose en partant d'acide gluconique y sont décrits. Un progrès a été accompli par V. Bilik (CZ - 232647, (1983)) en utilisant les ions cuivriques (Cu (II)) comme catalyseurs. Des rendements de l'ordre de 70 % sont atteints après une purification laborieuse.

Des résultats identiques ont été obtenus récemment avec un mélange d'ions ferriques et ferreux comme catalyseurs (CZ - 279002, (1994)).

Enfin, dans des conditions particulières, le document EP-A 0.716.067 rapporte des rendements de 78 % en certains aldoses.

Durant une investigation large de la réaction de RUFF, la Demanderesse a découvert que les sels de cobalt ainsi que ceux de ruthénium et de nickel catalysaient la réaction d'un acide aldonique à n + 2 atomes de carbone avec l'eau oxygénée pour donner, de façon surprenante, un ose à n atomes de carbone et non, comme on pouvait s'y attendre, un ose à n + 1 atomes de carbone. Il y a donc perte de deux atomes de carbone par rapport à l'acide aldonique de départ.

En poursuivant ses investigations, la Demanderesse a découvert que les sels de cobalt, de ruthénium ou de nickel catalysaient également la réaction des acides uroniques, respectivement aldariques, avec l'eau oxygénée pour donner, de façon inattendue, un dialdose, respectivement un acide uronique.

De la même façon que précédemment, le procédé conforme à l'invention permet d'obtenir un dialdose à n atomes de carbone en partant d'un acide uronique à n + 2 atomes de carbone ou un acide uronique à n atomes de carbones en partant d'un acide aldarique à n + 2 atomes de carbone. Dans tous les cas, il y a perte de deux atomes de carbone par rapport à l'acide uronique ou l'acide aldarique de départ.

Ainsi, selon l'invention, le procédé de fabrication d'un aldose ou d'un dérivé d'aldose, à n atomes de carbone, est caractérisé par le fait qu'une solution aqueuse d'un sel d'un dérivé acide d'ose à n + 2 atomes de carbone comportant au moins un motif α-hydroxyacide, à l'exception de l'acide gluconique, est mise au contact de peroxyde d'hydrogène en présence d'au moins un sel d'un métal choisi dans le groupe constitué du cobalt, du nickel et du ruthénium.

Le procédé de l'invention met donc en oeuvre un sel d'un dérivé acide d'ose.

Dans la présente invention, on entend par dérivé acide d'ose, un acide mono- ou dicarboxylique dérivé d'un aldose. Cette définition englobe en particulier :
- les acides aldoniques, qui sont des acides monocarboxyliques dérivés des aldoses par remplacement du groupement aldéhyde par un groupement carboxy, tels que les acides maltobionique, lactobionique, glucoheptonique, mannonique, idonique, galactonique, lyxonique, xylonique, arabinonique et ribonique à l'exception de l'acide gluconique ;
- les acides uroniques comportant un motif α-hydroxyacide, qui sont des acides monocarboxyliques dérivés des aldoses par remplacement du groupement CH₂OH par un groupement carboxy et dans lesquels le groupement aldéhyde a été protégé, tels que les acides glucuronique, iduronique, xyluronique et arabinuronique ;
- les acides aldariques, qui sont des acides dicarboxyliques dérivés des aldoses par remplacement des deux groupements terminaux (CHO et CH₂OH) par des groupements carboxy, tels que les acides glucarique et xylarique.

Dans la présente invention, on entend par sel d'un dérivé acide d'ose, le dérivé acide sous forme libre, sous forme lactonisée ou sous forme de mélange de ces deux formes, sous forme de sels ou sous forme d'esters. Ainsi, par exemple les sels de calcium, de sodium ou les γ- et δ-lactones de ces dérivés acides d'oses conviennent parfaitement.

Les acides aldoniques sont obtenus de manière connue par oxydation de l'ose correspondant. Cette étape d'oxydation peut être conduite soit par voie chimique, soit par voie microbiologique.

La voie chimique préférée dans le cadre de l'invention consiste à oxyder l'ose à l'aide d'air ou d'oxygène en milieu alcalin et à l'aide de catalyseurs au palladium.

un procédé particulièrement préféré est celui qui a été décrit dans le document US-A 4.845.208, dont la Demanderesse est cessionnaire, qui consiste à utiliser comme catalyseur d'oxydation, du palladium fixé sur charbon actif et dopé au bismuth.

On peut également envisager d'oxyder l'ose par voie électrolytique ou à l'aide d'hypobromite. On peut encore oxyder l'ose par voie microbiologique à l'aide de *Gluconobacter* ou *d'Aspergillus*.

Les acides uroniques peuvent être obtenus, de manière connue, soit par voie microbiologique à l'aide de *Penicillium brevicompactum*, *d'Ustiulina vulgaris* ou *d'Acetobacter melanogenum*, soit par voie chimique par hydrolyse des acides polyglycuroniques.

Les acides aldariques peuvent être obtenus, de manière connue, par oxydation à l'air en présence de platine, des oses correspondants.

De préférence, le procédé de l'invention est mis en oeuvre, dans l'eau, avec une teneur en matière sèche en sel du dérivé acide d'ose comprise entre 1 et 60 %, préférentiellement entre 5 et 50 et plus particulièrement entre 10 et 30 %.

Les contraintes de matière sèche inférieure sont imposées pour des raisons évidentes d'économie d'évaporation de l'eau et de réduction de la taille des réacteurs.

Les contraintes de matière sèche supérieure sont essentiellement imposées par des problèmes de solubilité ou de viscosité du milieu réactionnel.

Dans la présente description, tous les pourcentages sont exprimés par rapport au dérivé acide d'ose (exemple : 50 mol% signifie 50 moles de X pour 100 moles de dérivé acide d'ose, et 50 % signifie 50 g de X pour 100 g de dérivé acide d'ose au départ).

Dans le procédé conforme à l'invention, le catalyseur est constitué par des ions cobalt, ruthénium ou nickel qui peuvent être amenés sous forme d'un sel quelconque de cobalt, de ruthénium ou de nickel. Avantageusement, on préfère les sels de cobalt : acétate, acétylacétonate, halogénures, nitrate, sulfate, de cobalt par exemple conviennent parfaitement.

Une quantité de catalyseur comprise entre 0,001 et 50 %, préférentiellement entre 0,002 et 20 % et plus particulièrement entre 0,005 et 5 par rapport au sel du dérivé acide d'ose mis en oeuvre donne de bons résultats dans le procédé conforme à l'invention, tant en ce qui concerne le rendement que la pureté de l'aldose ou du dérivé d'aldose.

Au mélange de sel de dérivé acide d'ose, de catalyseur et d'eau ainsi réalisé, on ajoute alors lentement sous agitation, le peroxyde d'hydrogène, de préférence sous forme d'eau oxygénée d'une richesse de 30 %, à raison de 1 à 500 mol%, préférentiellement de 50 à 400 mol% et plus particulièrement de 100 à 300 mol% par rapport au sel de l'acide gluconique mis en oeuvre.

Il est possible d'utiliser du peroxyde d'hydrogène sous forme d'eau oxygénée d'une richesse supérieure à 30 %, notamment par exemple jusqu'à 70 %.

L'addition de l'eau oxygénée est effectuée à une vitesse d'introduction celle que la température du milieu réactionnel ne s'élève pas, de préférence, au-delà de 50°C et plus particulièrement au-delà de 40°C. Ainsi, la vitesse d'introduction de l'eau oxygénée se situe généralement entre 30 minutes et deux heures.

De préférence, le procédé de l'invention est mis en oeuvre à une température comprise entre 0 et 100°C et préférentiellement entre 10 et 50°C.

Des températures inférieures amènent des temps de réaction trop longs et des températures supérieures, outre qu'elles nécessiteraient l'emploi de réacteurs résistants à la pression, conduiraient à une dégradation des produits de la réaction.

Les températures de 20 à 40°C sont donc particulièrement préférées dans le procédé de l'invention.

De préférence encore, le procédé de l'invention est mis en oeuvre à un pH compris entre 2 et 12, préférentiellement compris entre 5 et 8 et plus particulièrement compris entre 6 et 7.

L'invention sera mieux comprise au moyen des exemples qui suivent et qui ont pour seul but de mieux illustrer l'invention sans vouloir la réduire aux modes de réalisation expressément décrits et aux seuls dérivés acides d'ose mis en oeuvre.

Dans les exemples qui suivent, tous les résultats sont exprimés en pourcentage molaire.

### EXEMPLE 1 : Synthèse du D-thréose

Le galactonate de calcium pentahydrate (130,1g/ 0,25 mole), le chlorure de cobalt hexahydrate (0,23g/ 0,98mmole) et de l'eau (1000ml) sont introduits dans un réacteur double enveloppe. Le mélange est porté à une température de 35°C et à un pH de 6,5 par addition de soude 2N. L'eau oxygénée à 35 % (129ml/1,5moles) est introduite en 75 minutes en maintenant la température entre 35 et 40°C et le pH à 6,5 à l'aide de soude 2N. A la fin de l'addition, la solution est agitée une heure supplémentaire. Le pH est amené à 2,5 par addition d'acide sulfurique concentré (14ml) afin de précipiter les sels de calcium. Après filtration, la solution rose a la composition suivante :
D-thréose : 85 %,
acide galactonique : 7 %.

### EXEMPLE 2 : Synthèse du D-arabinose

Le glucoheptonate de sodium dihydrate (71,3g/ 0,25mole), le chlorure de cobalt hexahydrate (0,12g/ 0,50mmole) et de l'eau (500ml) sont' introduits dans un réacteur double enveloppe. Le mélange est porté à une température de 30°C et à un pH de 6,5 par addition de soude 2N. L'eau oxygénée à 30 % (77ml/0,75mole) est introduite en 75 minutes en maintenant la température entre 30 et 35°C et le pH à 6,5 à l'aide de soude 2N. A la fin de l'addition, la solution est agitée une heure supplémentaire. Le pH est amené à 2,5 par addition d'acide sulfurique concentré (8m1). La solution rose a la composition suivante :
D-arabinose : 71,9 %,
acide glucoheptonique : 11,7 %.

### EXEMPLE 3 : Synthèse du D-glycéraldéhyde

L'arabinonate de sodium (47,9g/0,255mole), le chlorure de cobalt hexahydrate (1,03g/4,33mmoles) et de l'eau (500ml) sont introduits dans un réacteur double enveloppe. Le mélange est porté à une température de 25°C et à un pH de 6,5 par addition de soude 2N. L'eau oxygénée à 30 % (55ml/0,54mole) est introduite en 75 minutes en maintenant la température entre 25 et 30°C et le pH à 6,5 à l'aide de soude 2N. A la fin de l'addition, la solution est agitée une heure supplémentaire. Le pH est amené à 2,5 par addition d'acide sulfurique concentré (14ml). La solution rose a la composition suivante :
D-glycéraldehyde : 30 %,
acide arabinonique : 43 %,
acide érythronique : 9 %.

### EXEMPLE 4 : Synthèse du glucosyl-1,2-érythrose

La maltobionate de calcium (75,2g / 0,1mole), le chlorure de cobalt hexahydrate (0,38g / 1,6mmoles) et de l'eau (300ml) sont introduits dans un réacteur double enveloppe. Le mélange est porté à une température de 30°C et à un pH de 6,5 par addition de soude 2N. L'eau oxygénée à 35 % (52ml / 0,6mole) est introduite en 75 minutes en maintenant la température entre 30 et 35°C et le pH à 6,5 à l'aide de soude 2N. A la fin de l'addition, la solution est agitée une heure supplémentaire. Le pH est amené à 2,5 par addition d'acide sulfurique concentré (14ml) afin de précipiter les sels de calcium. Après filtration, la solution rose contient le glucosyl-1,2-érythrose avec un rendement molaire de 85 %.

### EXEMPLE 5 : Synthèse des acides tétruroniques

Le glucarate de potassium monosel (63,2g/ 0,255mole), le chlorure de cobalt hexahydrate (1,3g/ 5,46mmoles) et de l'eau (500ml) sont introduits dans un réacteur double enveloppe. Le mélange est porté à une température de 25°C et à un pH de 6,5 par addition de soude 2N. L'eau oxygénée à 30 % (55ml/0,54mole) est introduite en 75 minutes en maintenant la température entre 25 et 30°C et le pH à 6,5 à l'aide de soude 2N. A la fin de l'addition, la solution est agitée une heure supplémentaire. Le pH est amené à 2,5 par addition d'acide sulfurique concentré (14ml). La solution rose a la composition suivante :
acides tétruroniques (thréuronique et érythruronique) : 44 %,
acide glucarique : 23 %.

## Revendications

1. Procédé de fabrication d'un aldose ou d'un dérivé d'aldose, à n atomes de carbone, caractérisé par le fait qu'une solution aqueuse d'un sel d'un dérivé acide d'ose à n + 2 atomes de carbone comportant au moins un motif α-hydroxyacide, à l'exception de l'acide gluconique, est mise au contact de peroxyde d'hydrogène en présence d'au moins un sel d'un métal choisi dans le groupe constitué du cobalt, du nickel et du ruthénium.

2. Procédé de fabrication selon la revendication 1, caractérisé par le fait que la solution aqueuse a une matière sèche en sel du dérivé acide d'ose à n + 2 atomes de carbone comportant au moins un motif α-hydroxyacide comprise entre 1 et 60 %.

3. Procédé de fabrication selon la revendication 1 ou 2, caractérisé par le fait que le sel de métal est présent en une quantité comprise entre 0,001 et 50 % exprimée par rapport au sel du dérivé acide d'ose à n + 2 atomes de carbone comportant au moins un motif α-hydroxyacide.

4. Procédé de fabrication selon l'une quelconque des revendications 1 à 3, caractérisé par le fait que l'on utilise le peroxyde d'hydrogène de préférence sous forme d'eau oxygénée d'une richesse de 30 %, en une quantité comprise entre 1 et 500 mol% exprimée par rapport au sel du dérivé acide d'ose à n + 2 atomes de carbone comportant au moins un motif α-hydroxyacide.

5. Procédé de fabrication selon l'une quelconque des revendications 1 à 4, caractérisé par le fait que l'on travaille à une température comprise entre 0 et 100°C et de préférence entre 10 et 50°C.

6. Procédé de fabrication selon l'une quelconque des revendications 1 à 5, caractérisé par le fait que l'on travaille à un pH compris entre 2 et 12, et de préférence entre 5 et 8.

7. Procédé de fabrication d'un aldose selon l'une quelconque des revendications 1 à 6, caractérisé par le fait que le sel d'un dérivé acide d'ose à n + 2 atomes de carbone comportant au moins un motif α-hydroxyacide est un sel d'acide aldonique.

8. Procédé de fabrication d'un dialdose selon l'une quelconque des revendications 1 à 6, caractérisé par le fait que le sel d'un dérivé acide d'ose à n + 2 atomes de carbone comportant au moins un motif α-hydroxyacide est un sel d'acide uronique.

9. Procédé de fabrication d'un acide uronique selon l'une quelconque des revendications 1 à 6, caractérisé par le fait que le sel d'un dérivé acide d'ose à n + 2 atomes de carbone comportant au moins un motif α-hydroxyacide est un sel d'acide aldarique.

## Patentansprüche

1. Verfahren zur Herstellung von Aldosen oder Aldosederivaten mit n Kohlenstoffatomen, dadurch gekennzeichnet, dass eine wässrige Lösung eines Salzes eines sauren Osederivats mit n + 2 Kohlenstoffatomen, das mindestens eine a-Hydroxy-säure-Einheit aufweist, Gluconsäure ausgenommen, in Gegenwart von mindestens einem Salz eines Metalls, das aus der aus Kobalt, Nickel und Ruthenium bestehenden Gruppe ausgewählt ist, mit Wasserstoffperoxid in Kontakt gebracht wird.

2. Verfahren nach Anspruch 1, dadurch gekennzeichnet, dass die wässrige Lösung einen Trockenmassegehalt an Salz des sauren Osederivats mit n + 2 Kohlenstoffatomen, das mindestens eine α-Hydroxysäure-Einheit aufweist, von 1 bis 60 % besitzt.

3. Verfahren nach Anspruch 1 oder 2, dadurch gekennzeichnet, dass das Metallsalz in einer Menge von 0,001 bis 50 %, bezogen auf das saure Osederivat mit n + 2 Kohlenstoffatomen, das mindestens eine α-Hydroxysäure-Einheit aufweist, vorliegt.

4. Verfahren nach einem der Ansprüche 1 bis 3, dadurch gekennzeichnet, dass man Wasserstoffporoxid vorzugsweise in Form einer 30 %igen wässrigen Lösung in einer Menge von 1 bis 500 Mol%, bezogen auf das Salz des sauren Osederivats mit n + 2 Kohlenstoffatomen, das mindestens eine α-Hydroxysäure-Einheit aufweist, verwendet.

5. Verfahren nach einem der Ansprüche 1 bis 4, dadurch gekennzeichnet, dass man bei einer Temperatur von 0 bis 100°C und vorzugsweise von 10 bis 50°C arbeitet.

6. Verfahren nach einem der Ansprüche 1 bis 5, dadurch gekennzeichnet, dass man bei einem pH von 2 bis 12, vorzugsweise von 5 bis 8, arbeitet.

7. Verfahren zur Herstellung von Aldosen nach einem der Ansprüche 1 bis 6, dadurch gekennzeichnet, dass das Salz eines sauren Osederivats mit n + 2 Kohlenstoffatomen, das mindestens eine α-Hydroxysäure-Einheit aufweist, ein Aldonsäuresalz ist.

8. Verfahren zur Herstellung von Dialdose nach einem der Ansprüche 1 bis 6, dadurch gekennzeichnet, dass das Salz eines sauren Osederivats mit n + 2 Kohlenstoffatomen, das mindestens eine α-Hydroxysäure-Einheit aufweist, ein Uronsäuresalz ist.

9. Verfahren zur Herstellung von Uronsäure nach einem der Ansprüche 1 bis 6, dadurch gekennzeichnet, dass das Salz eines sauren Osederivats mit n + 2 Kohlenstoffatomen, das mindestens eine α-Hydroxysäure-Einheit aufweist, ein Aldarsäuresalz ist.

## Claims

1. Method of manufacturing an aldose or an aldose derivative, with n carbon atoms, characterised by the fact that an aqueous solution of a salt of an acid derivative of an ose with n + 2 carbon atoms containig at least one α-hydroxy acid unit, with the exception of gluconic acid, is brought into contact with hydrogen peroxide in the presence of at least one salt of a metal chosen from the group consisting of cobalt, nickel and ruthenium.

2. Method of manufacture according to claim 1, characterised by the fact that the aqueous solution has a salt dry matter of the acid derivative of the ose with n + 2 carbon atoms containing at least one α-hydroxy acid unit of between 1 and 60%.

3. Method of manufacture according to claim 1 or 2, characterised by the fact that the metal salt is present in a quantity of between 0.001 and 50% expressed in relation to the salt of the acid derivative of the ose with n + 2 carbon atoms containing at least one α-hydroxy acid unit.

4. Method of manufacture according to any one of claims 1 to 3, characterised by the fact that hydrogen peroxide is used, preferably in the form of oxygenated water with a strength of 30%, in a quantity of between 1 and 500 mol % expressed in relation to the salt of the acid derivative of the ose with n + 2 carbon atoms containing at least one α-hydroxy acid unit.

5. Method of manufacture according to any one of claims 1 to 4, characterised by the fact that the process is carried out at a temperature of between 0 and 100°C and preferably between 10 and 50°C.

6. Method of manufacture according to any one of claims 1 to 5, characterised by the fact that the process is carried out at a pH of between 2 and 12, preferably between 5 and 8.

7. Method of manufacturing an aldose according to any one of claims 1 to 6, characterised by the fact that the salt of an acid derivative of the ose with n + 2 carbon atoms containing at least one α-hydroxy acid unit is a salt of aldonic acid.

8. Method of manufacturing a dialdose according to any one of claims 1 to 6, characterised by the fact that the salt of an acid derivative of the ose with n + 2 carbon atoms containing at least one α-hydroxy acid unit is a salt of uronic acid.

9. Method of manufacturing a uronic acid according to any one of claims 1 to 6, characterised by the fact that the salt of an acid derivative of the ose with n + 2 carbon atoms containing at least one α-hydroxy acid unit is a salt of aldaric acid.
